(19) **Europäisches Patentamt**
**European Patent Office**
**Office européen des brevets**

(11) Publication number: **0 408 146 A2**

## EUROPEAN PATENT APPLICATION

(21) Application number: **90201870.4**

(22) Date of filing: **11.07.90**

(51) Int. Cl.5: **C12P 21/02, C07K 13/00, A61K 37/24, //C12N15/16**

(30) Priority: **13.07.89 US 379298**

(43) Date of publication of application:
**16.01.91 Bulletin 91/03**

(84) Designated Contracting States:
**CH DE FR GB IT LI NL**

(71) Applicant: **MERCK & CO. INC.NC.**
**126, East Lincoln Avenue P.O. Box 2000**
**Rahway New Jersey 07065-0900(US)**

(72) Inventor: **Yamazaki, Shigeko**
**1279 Schwab Road**
**Hatfield, NJ 19440(US)**
Inventor: **De Phillips, Peter A.**
**577 Hidden Valley Road**
**King of Prussia, Pennsylvania 19406(US)**

(74) Representative: **Hesketh, Alan, Dr. et al**
**European Patent Department Merck & Co.,**
**Inc. Terlings Park Eastwick Roadoad**
**Harlow Essex, CM20 2QR(GB)**

(54) **Method for the purification of therapeutically active recombinant acidic fibroblast growth factor.**

(57) An improved method for the purification of acidic fibroblast growth factor (aFGF) is disclosed. The methodology includes extraction, ion exchange chromatography, affinity chromatography, reversed phase-HPLC, solvent exchange solubilization and/or hydrophobic interaction chromatography. The method results in an increase in mitogenic potency, an increase in solubility and allows the separation of recombinant aFGF from truncated forms of recombinant aFGF.

FIG. 1A

FIG. 1B

FIG. 1C

FIG. 1D

BRIEF DESCRIPTION OF THE DRAWINGS

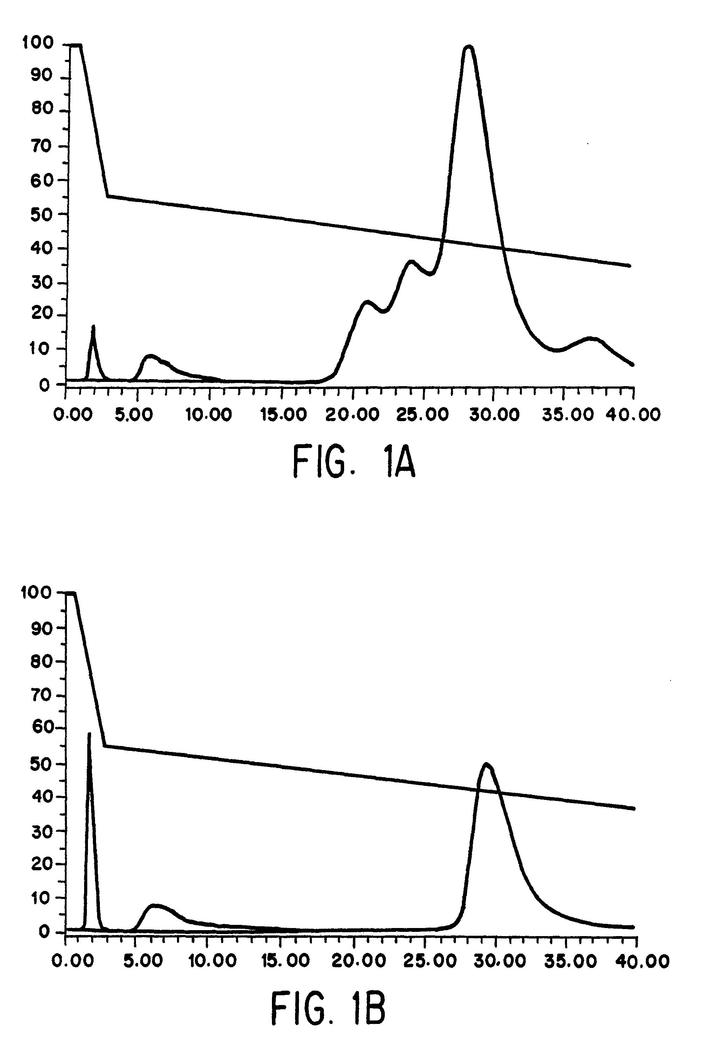

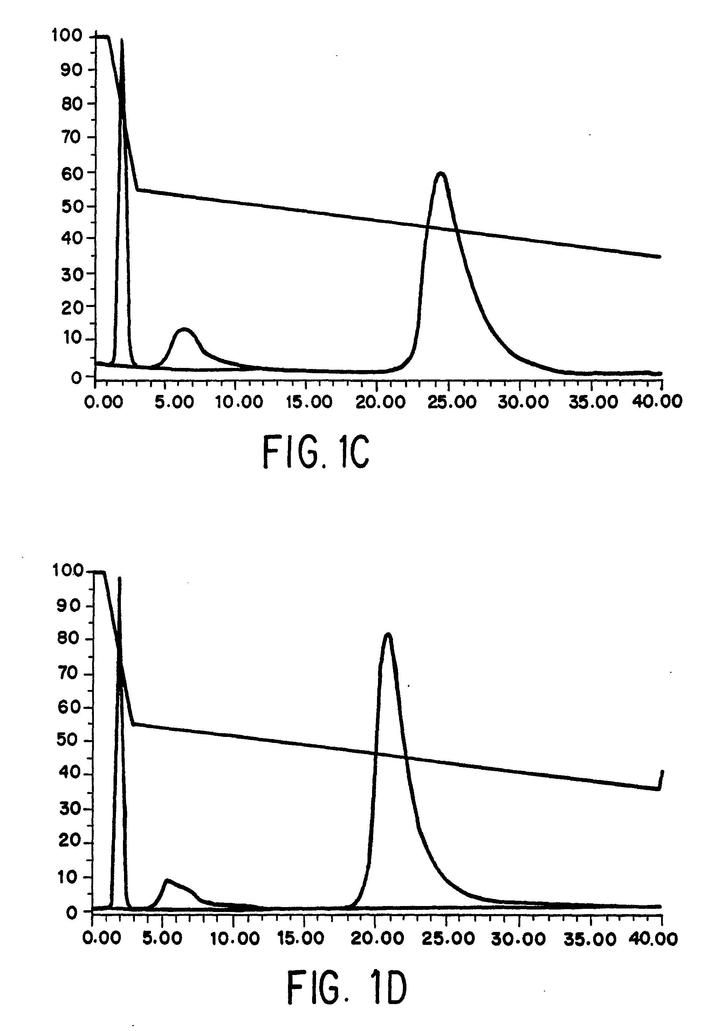

Figure 1 depicts the HIC separation profiles of:
a) combined sample containing full length and truncated r-haFGF;
b) individual full length r-haFGF;
c) individual truncated r-haFGF with the first amino acid removed from the amino terminus;
d) individual truncated r-haFGF with the first two amino acids removed from the amino terminus.

BACKGROUND OF THE INVENTION

Acidic fibroblast growth factor was first purified to homogeneity by a multistep process which included extraction and differential salt precipitation, carboxymethyl-Sephadex chromatography, gel filtration, carboxymethyl-Sephadex chromatography, isoelectric focusing and reverse-phase high performance liquid chromatography (HPLC), Thomas, U.S. Patent No. 4,444,760. Recombinant aFGF (r-aFGF) has been purified to homogeneity by a simple two-step chromatographic procedure employing a combination of heparin-Sepharose affinity chromatography followed by reversed-phase HPLC, European Pat. Appl., Pub. No. 259,953.

The present invention provides an improved process for the purification of highly concentrated recombinant derived acidic fibroblast growth factor which results in a 5-fold increase in mitogenic potency and a 170 fold increase in solubility in a dosing buffer with recovery greater than 90%. This allows for the formulation of a higher dosage of r-aFGF. The process also allows the separation of truncated forms of r-aFGF from the full length form.

OBJECT OF THE INVENTION

It is accordingly, an object of the present invention is to provide an efficient procedure for the purification of large batches of recombinant derived human acidic fibroblast growth factor. Another object is to provide a highly concentrated bulk solution of active protein in dosing buffer prior to final formulation. A further object is to provide a rapid and easy procedure for the separation of truncated forms of aFGF from the full length form. Another object is to provide glycosaminoglycan stabilized aFGF.

SUMMARY OF THE INVENTION

An improved method for the purification of acidic fibroblast growth factor (aFGF) is disclosed. The methodology includes extraction, ion exchange chromatography, affinity chromatography, reversed-phase-HPLC, solvent exchange solubilization and hydrophobic interaction chromatography. The method results in an increase in mitogenic potency, an increase in solubility and allows the separation of recombinant aFGF from truncated forms of recombinant aFGF.

DETAILED DESCRIPTION OF THE INVENTION

The present invention is useful for the purification of recombinant acidic fibroblast growth factor (aFGF) produced in either procaryotic or eucaryotic cells. Eucaryotic cell types include yeast cells and mammalian cells. Procaryotic cell types include bacterial cells with Eschercia coli being preferred.

Recombinant derived aFGF is obtained using standard recombinant methodology such as that described in European Pat. Appl., Pub. No. 259,953. Transformed cells producing recombinant aFGF are grown using standard procedures for the specific cell type. Acidic aFGF is obtained by collection of the cell culture fluid or by mechanical or chemical disruption of washed cells, with mechanical disruption of the cells being preferred. The generally soluble aFGF is diluted to minimize association of impurities with aFGF that may prevent binding to the matrix in the initial ion exchange chromatographic process.

Isolation and initial purification of aFGF present in the diluted cell-free extract is carried out by ion exchange chromatography. It is generally preferred that a weak cationic exchanger such as CM Sephadex C-50, CM Sepharose Fast Flow and S Sepharose Fast Flow (Pharmacia) or Bio-Rex 70 (Bio-Rad) be used for the isolation of aFGF. The preferred resin is CM Sephadex C-50. Isolation is carried out by loading the sample onto a large CM Sephadex C-50 column, eluting the impurities with about 10 mM EDTA in phosphate buffered saline (PBS) and eluting aFGF with about 0.6 M NaCl, 10 mM EDTA in PBS. A significant improvement over previous methods of isolation was effected by increasing the amount of resin and switching from a batch adsorption technique to a column technique. The improvement in recovery is greater than 3-fold.

Further purification of isolated aFGF is carried out using heparin or heparin analog affinity resins. Examples of affinity resins include, but are not limited to, Heparin-Sepharose, Heparin-Sepharose Fast Flow (Pharmacia), TSK Gel Heparin 5 PW (Toso-Haas) and Cellfine-Sulfate (Amicon Co.), with Heparin-Sepharose being preferred. Acidic aFGF eluted from the CM Sephadex column is applied to a Heparin-Sepharose column and the column is washed with about 0.8 M NaCl in PBS. The bound product is eluted from the column with about 1.5 M NaCl in PBS.

Typically, the affinity-purified aFGF is further purified by preparative reverse-phase high performance liquid chromatography (RP-HPLC) using a C$_4$ matrix such as Dyanamax or Vydac, with Dyanamax being preferred. The sample from the affinity column is applied on a preparative column and eluted with a gradient of CH$_3$CN about (0-60%) in 0.1% trifluoroacetic acid (TFA). This step removes trace impurities such as DNA and lipopolysaccharides and results in about 90% recovery.

When concentrations of the RP-HPLC product higher than about 50 µg/ml are introduced into an aqueous formulation buffer such as PBS, the purified aFGF forms aggregates which can increase in size until the aFGF precipitates out of solution. Aggregation can also result in a loss of aFGF because the aggregates adhere to containers used in the processing.

Aggregation is prevented and the aFGF is maintained in a soluble biologically active state by solvent exchange procedures. Solvent exchange as used herein is defined as the controlled exchange of the RP-HPLC elution solvent for an aqueous solvent under conditions which prevent the formation of aggregates of aFGF. Procedures in which solvent exchange may be used include, but are not limited to, dialysis, diafiltration, ultrafiltration and affinity chromatography with dialysis being preferred. The RP-HPLC eluate, which is generally aFGF in acetonitrile/trifluoroacetic acid, is diluted to about 2 mg protein per ml solution by adding pyrogen-free water. The diluted solution is then dialyzed against an intermediate solution followed by dialysis against a dosing buffer solution. The intermediate solution generally contains a chaotropic agent which in aqueous solution and under suitable concentrations is capable of changing the spatial configuration of proteins. Examples of such agents include, but is not limited to, urea, guanidine hydrochloride, sodium thiocyanate and detergents such as sodium dodecylsulfate, with guanidine hydrochloride being preferred. Sugars such as sucrose or maltose can be used as an intermediate solution (1 M) instead of chaotropic agents. The intermediate solution may also contain reducing agents known to reduce disulfide bonds such as, but not limited to, mercaptoethanol, dithiothreitol (DTT), dithioerythritol, and cystine, with DTT being preferred. The preferred intermediate solution contains about 2 M to about 5 M guanidine hydrochloride with about 0.1 mM to about 10 mM DTT and the dialysis is carried out from about 2 to about 16 hours with about 7 to about 11 hours preferred. The volumes range from about 10 to about 500 volumes of the intermediate solution. The aFGF is further dialyzed against a dosing buffer such as PBS for from about 1 to about 3 days with about 2 to about 6 x 100 sample volumes of PBS. Following dialysis against PBS the resulting concentration of about 1.5 to about 2.5 mg/ml solution in PBS is achieved. Acidic fibroblast growth factor which has been dialyzed is further concentrated to about 12 mg/ml solution in PBS by ultrafiltration. It is preferred that ultrafiltration take place in an Amicon cell with a YM membrane, either YM 10 or YM30, with YM 30 being preferred.

The solvent exchange process is scaled-up for the production of large amounts of non-precipitating aFGF. A continuous crossflow dialysis cartridge is substituted for the dialysis tubing for the scale-up process. The cartridge is actually two containers separated by a semipermeable membrane. The cartridge of choice is a hollow-fiber cellulose membrane cartridge (Enka AG). The eluate from the RP-HPLC column is diluted with pyrogen-free water to about 2mg/ml and the sample is pumped through the hollow-fibers while the dialysate is circulated on the other side of the fibers, thus allowing dialysis to take place. Both fluids are pumped by separate pumps. The solvent exchange process as described above is modified to enhance the performance of the crossflow dialysis system. The quantity of guanidine hydrochloride is minimized by the use of pyrogen-free water (first solution), about 5 to about 20 volumes, as the first dialyzing solution to remove the majority of the organic eluant. The aFGF is now dialyzed against guanidine hydrochloride, about 2 M to about 5 M with about 0.1 mM to about 10 mM DTT, with about 3M guanidine hydrochloride and about 5 mM DTT being preferred, about 1 to about 10 volumes. This second solution was dialyzed against the aFGF for about 5 minutes to about 1 hour, with about 25 minutes being preferred. A third solution consisting of about 0.05 M to about 0.5 M guanidine hydrochloride, about 2 to about 10 volumes, was dialyzed against the aFGF for from about 5 minutes to about 1 hour. The aFGF was then dialyzed against PBS, about 10 to about 40 volumes for from about 5 minutes to about 1 hour. All solutions are used at a pH of from about pH 6.0 to about pH 8.0 and the dialysis solution was circulated at a rate of from about 1 ml/min to about 50 ml/min. This multistep process results in yields of r-aFGF that are more than sufficient for the formulation of r-aFGF as pharmacological agent for tissue repair.

Recombinant derived acidic fibroblast growth factor expressed in E. coli by a modification of the technique described in European Pat. Appl., Pub. No. 259,953 may result in a heterogenous preparation containing both full-length r-aFGF and truncated forms of r-aFGF when the transformed cells are grown under certain conditions. Truncated as used herein refers to a protein which is lacking one or more of the expected or normal terminal amino acids. When the protein is purified through the Heparin-Sepharose step, the concentrations may range from about 65%-75% full-length r-aFGF and about 25%-35% truncated r-aFGF as determined by hydrophobic interaction (HI) chromatography (HIC). Hydrophobic interaction chromatography may be carried out using conventional liquid column chromatography procedures or high performance liquid chromatography procedures. Conventional HIC resins include, but are not limited to, Butyl-Agarose (Pharmacia), Hexyl-Agarose (Agarose Hexane, Pharmacia) and Octyl-Agarose (Pharmacia) with Butyl-Agarose being preferred. Standard chromatography procedures with these resins maintains the integrity of the proteins and they are highly soluble in dosing buffer. Convential HIC can be substituted for RP-HPLC without the need for solvent exchange to maintain the solubility of r-aFGF.

Examples of HI-HPLC resins include, but are not limited to, Hydropore-HIC (Rainin), Spherogel-HIC (Beckman), TSK gel Ether-5PW (TosoHass), with TSK gel Ether-5PW and Hydropore-HIC being preferred. The Heparin-Sepharose product is applied on a Hydropore-HIC column (Rainin) and eluted with a reversed salt gradient of about 2 to about 1 M $(NH_4)_2 SO_4$ in PBS. The truncated forms of r-aFGF elute ahead of the full-length r-aFGF indicating that full-length r-aFGF is more hydrophobic in nature than the truncated forms. The r-aFGF is insoluble upon dialysis against PBS and requires solvent exchange by the above procedure prior to dosing formulation. HI-HPLC can replace RP-HPLC in the purification process but requires the solvent exchange step to maintain a soluble product.

The biological activity of the purified r-aFGF is evaluated using a fibroblast mitogenic assay modified from Thomas et al ., J. Biol. Chem. 225 : 5517-5520 (1980). The heat-inactivated calf serum of the prior technique is replaced with about 1% insulin-selenium-transferin (ITS), about 0.4 gm L-histidine, 50 $\mu$l of 1 M ethanolamine, 1.25 gm bovine serum albumin with 5.35 mg of linoleic acid per liter of about 75% Delbucco's modified Egale's medium, about 25% Ham's F12 containing both penecillin-streptomycin and L-glutamine.

Highly purified, soluble r-aFGF loses biological activity and exhibits a shortened shelf life when stored at temperatures above freezing for extended periods of time. This is disadvantageous because such loss of activity due mainly to precipitation or aggregation makes it impractical to store preparations of r-aFGF for the periods of time necessary for a wound-healing product. The present invention provides a means of reducing the loss of biological activity during storage. Thermal instability can be prevented by the addition of glycosaminoglycans to the r-aFGF. Glycosaminoglycans are polysaccharide chains found in prot-eoglycans and include, but are not limited to, hyaluronate, chondroitin sulfate, keratan sulfate, heparan sulfate, and heparin. Heparin is the preferred stabilizing agent. The heparin may be prepared as the sodium, potassium, ammonium, barium, calcium or magnesium salt, with the sodium salt preferred. Acidic fibroblast growth factor is stabilized by the addition of heparin sodium, about 0.01 mg/ml to about 10.0 mg/ml with about 0.01 mg/ml to about 1.5 mg/ml being preferred. The presence of heparin in r-aFGF stored at 4°C prevents any loss of biological activity by maintaining the r-aFGF in a highly soluble state. The stabilizing effect of heparin is even more striking when the r-aFGF is stored at 25°C. The heparin stabilized r-aFGF lost about 20% activity while the non-stabilized control lost 100% activity.

Stabilized r-aFGF is useful for topical wound healing or tissue repair. For topical application, various pharmaceutical formulations are useful for the administration of stabilized r-aFGF. Such formulations include, but are not limited to the following: ointments such as hydrophilic petrolatum or polyethylene glycol ointment; pastes which may contain gums such as xanthan gum; gels such as aluminum hydroxide or sodium alginate gels; albumins such as human or animal albumins; collagens such as human or animal collagens; celluloses such as alkyl celluloses, hydroxyalkyl celluloses and alkylhydroxyalkyl celluloses, for example methylcellulose, hydroxyethyl cellulose, carboxymethyl cellulose, hydroxypropyl methylcellulose, and hydroxypropyl cellulose; poloxamers such as Pluronic® Polyols exemplified by Pluronic F-127; tetronics such as tetronic 1508; and alginates such as sodium alginate. The pharmaceutical formulation will include solubilized and stabilized r-aFGF in amounts of about 0.1 to about 100 $\mu$g/ml.

The following examples illustrate the present invention without, however, limiting the same thereto.

EXAMPLE 1

### Extraction

All purification steps were performed at 4°C except the HPLC steps. Frozen cell paste (700 g) of recombinant human aFGF (r-haFGF) expressed in E. coli cells was suspended in 2.8 L of 0.12 M NaCl, 10 mM EDTA, with or without 1 mM PMSF in 6.2 mM sodium phosphate, pH 7.2 (cell breakage buffer), and the cells were disrupted by two passes through a Laboratory Homogenizer (Gaulin Corporation) equipped with cooling system, at a pressure of 8,000-9,000 psi. The cell-lysate was diluted by adding the cell-breakage buffer (2.8 L) to eliminate association of impurities with the product. The diluted cell-lysate was centrifuged at 4,000 rpm (4,000 X g) for twenty minutes using a Beckman centrifuge (Model J-6M/E) to remove cell debris. The resulting supernatant is the cell-free extract which contains most of the aFGF (>95%, about 12 g aFGF) present in the E. coli cells (Table 1). The concentration of aFGF was determined by RP HPLC on samples treated with guandine-HCl.

### EXAMPLE 2

### CM Sephadex Chromatography

The product (12 g) present in the cell-free extract from Example 1, was initially purified by loading the sample on a CM Sephadex C-50 column (25.2 X 30 cm) which was pre-equilibrated with 0.12 M NaCl, 10 mM EDTA in 6.2 mM sodium phosphate, pH 7.2. After completion of sample loading, the impurities were eluted from the column with 40 L of 0.12 M NaCl, 10 mM EDTA in 6.2 mM sodium phosphate, pH 7.2. The bound growth factor (7.7 g) was eluted from the column as a single peak with 0.6 M NaCl, 10mM EDTA in 6.2 mM sodium phosphate, pH 7.2. The mitogenesis assay of the product indicated that the growth factor was in the biologically active state ($ED_{50} = 250$ pg/ml). SDS-PAGE silver stain showed the presence of small amounts of impurities (Mr<10,000) in the product (>90% purity). Most of the impurities including proteins, DNA and lipopolysaccharide (LPS) were removed by this chromatography step (Table 1).

### TABLE 1

| PURIFICATION OF r-haFGF FROM E. Coli | | | | | | |
|---|---|---|---|---|---|---|
| Sample | aFGF | | Protein (g) | DNA (mg) | LPS EU | Mitogensis $ED_{50}$ (pg/ml) |
| | Total Recovery | | | | | |
| | (g) | (%) | | | | |
| Crude cell lysate | 12.5 | 100 | 109 | 3,700 | $4.8 \times 10^{11}$ | -- |
| Cell-free extract | 11.6 | 93 | 94 | 3,400 | $4.6 \times 10^{11}$ | -- |
| CM Sephadex | 7.7 | 62 | 15 | <13 | $1.6 \times 10^{5}$ | 250 |
| Heparin Sepharose | 7.5 | 61 | 14 | <13 | 891 | 141 |
| RP-HPLC | 4.5 | 36 | -- | <13 | 23 | 1000 |

### EXAMPLE 3

## Heparin-Sepharose Chromatography

The CM Sephadex eluate, from Example 2, (7.7 g r-haFGF) was loaded onto a Heparin-Sepharose column (11.3 x 13 cm) which was pre-equilibrated with 0.6M NaCl, 10 mM EDTA in 6.2 mM sodium phosphate, pH 7.2, at flow rate of 20 ml/min. The unadsorbed material was washed by elution with 0.8 M NaCl in 6.2 mM sodium phosphate, pH 7.2. The bound growth factor was eluted as a single peak by 1.5 M NaCl in 6.2 mM sodium phosphate, pH 7.2. The yield of r-haFGF (7.5 g aFGF) was 97% and LPS was reduced 180-fold. The mitogenic activity of the affinity-purified product was $ED_{50} = 141$ pg/ml, see TABLE 1, and the high solubility of the growth factor in PBS (5 mg/ml) indicated that the product was in a highly active state.

## EXAMPLE 4

## Preparative Reversed-Phase HPLC

The heparin-Sepharose purified product, from Example 3, (1.0 g of r-haFGF) was loaded onto a preparative C4-RP HPLC column (Dynamax C4 300 Angstrom 12 micron 4.14 X 25 cm) which was equilibrated with 12% $CH_3CN$ in 0.1% trifluoroacetic acid (TFA). The column was eluted at flow rate 20 ml/min with a linear gradient of 12-24% $CH_3CN$ in 0.1% TFA for eight minutes followed by a linear gradient of 24-60% $CH_3CN$ in 0.1% TFA for 36 minutes. The growth factor was eluted as a single peak (0.8 g) with the LPS being decreased 39-fold. The r-haFGF became aggregated during this process and required deaggregation to enhance solubility of the product in PBS.

## EXAMPLE 5

## Solvent Exchange

The RP-HPLC purified product (0.18g in $CH_3CN$/TFA), from Example 4, was diluted with water to a concentration of 2.4 mg/ml (75 ml), and placed in a dialysis cellulose bag (6,000-8,000 MW cut off, Spectrum Medical Industries, Inc.). The material was dialyzed against 2 X 3.5 L of 3 M guanidine-HCl in 5 mM DTT over a nine-hour period, followed by a second dialysis against 4 X 7 L of PBS over a two-day period in a capped bottle. The resulting dialysate, containing some precipitate, was centrifuged for five minutes at 2,000 rpm in a IEC Centrifuge Model DPR-600 (Damon/IEC) to remove precipitate. The supernatant was then filtered through a 0.2 micron Sterivex-GV filter unit (Millipore). The non-aggregated aFGF (0.17 g), after filtration, exhibited a 90% recovery and a concentration of 2 mg/ml in PBS and was biologically active ($ED_{50} = 126$ pg/ml).

As an alternative to the use of a dialysis tubing for the prevention of aggregation of r-haFGF by two-step dialysis, a rapid scalable procedure was developed using an Enka Crossflow dialysis system. This system uses a hollow-fiber cellulose membrane cartridge (10,000 MW cut off) and two pumps. A solution of aFGF (1.6 mg/ml, 0.27 g) in acetonitrile/TFA was circulated through the inside of the hollow fiber capillary, while on the outside dialysis solution was circulated once in the opposite direction.

The two-step dialysis was modified to enhance the performance of the process in the cartridge (Table 2). An initial single pass of pyrogen-free water (1 L) was used to remove most of TFA/acetonitrile. Then a single pass of 3 M guanidine-HCl/5 mM DTT/10 mM sodium phosphate pH, 7.2 (0.5 L), was employed over a 25 minute period. To avoid precipitation of r-haFGF by rapid removal of guanidine-HCl, stepwise dialysis was carried out with 0.1 M guanidine-HCl in 10 mM sodium phosphate, pH 7.2 (1 L). Residual guanidine was removed by dialysis against a single pass of PBS (2 L). The dialyzed r-haFGF was filtered through a 0.2 $\mu$ filter. This scalable procedure yielded 0.26 g of soluble r-haFGF with high mitogenic activity ($ED_{50} = 224$ pg/ml).

6

TABLE 2

| SOLVENT EXCHANGE CONDITIONS FOR ENKA CROSSFLOW DIALYSIS CARTRIDGE | | | |
|---|---|---|---|
| Dialysis Solution | Volume (L) | Flow Rate ml/min | Time min |
| Pyrogen-free water | 1 | 50 | 20 |
| 3 M guanidine hydrochloride in 5 mM DTT | 0.5 | 20 | 25 |
| 0.1 M guanidine hydrochloride | 1 | 20 | 50 |
| PBS | 2 | 20 | 100 |

EXAMPLE 6

Hydrophobic Interaction HPLC

An affinity purified material (0.2 ml, 2.6 mg) known to contain about 10% truncated r-haFGF and about 90% full-length r-haFGF was applied to a Hydropore-HIC column (4.6 X 10 mm, particle size 12 micron, pore size 300 angstroms, Rainin Instrument Co.) which had been pre-equilibrated with 3 M $(NH_4)_2SO_4$ in PBS, at a flow rate of 1 ml/min. The column was eluted with a reverse salt gradient of 2-1 M $(NH_4)_2SO_4$ in PBS for 40 minutes (Figure 1). A small peak fraction containing truncated aFGF was eluted prior to the major peak which contained full-length r-haFGF (>99%). The results of N-terminal amino acid sequencing analysis of these fractions showed that the major peak, full-length r-haFGF, is free from truncated r-haFGF, and the smaller peak contained 79% truncated and 21% full-length r-haFGF. The results of the mitogenesis assay showed that both peak fractions contained biologically active growth factor.

EXAMPLE 7

Butyl-Agarose And Hexyl-Agarose Hydrophobic Interaction Chromatography

Heparin-Sepharose purified r-haFGF (165 mg) known to contain about 70% full-length r-aFGF, about 20% with a single amino acid missing and about 10% with two amino acids missing was mixed with an equal volume of 3 M $(NH_4)_2SO_4$. The mixture was applied to Butyl-Agarose column (2 x 5 cm, Pharmacia) which had been pre-equilibrated with 2 M $(NH_4)_2SO_4$ in PBS. The column was eluted with 400 ml of a reverse salt gradient 1.8-1 M $(NH_4)_2SO_4$ in PBS and the elution was monitored by U.V. absorption at 280 nm. The U.V. absorbing fractions were assayed by hydrophobic interaction HPLC as described in Example 6. The double truncate enriched fractions eluted first then the single truncate enriched fractions eluted prior to full-length r-haFGF fractions. The process resulted in 56 mg of full-length r-haFGF which was free of truncated forms of r-haFGF. Resolution of the Butyl-Agarose column chromatography was slightly less than that seen with HI-HPLC. The purified full-length r-haFGF that eluted from the column was soluble after removal of $(NH_4)_2SO_4$ by dialysis against PBS and when added to formulation buffer (PBS).

Heparin-Sepharose purified r-haFGF (110 mg), as described above, was chromatographed on a Hexyl-Agarose column (2.5 x 5 cm, Pharmacia, Hexamine) under conditions as described above. Again, full-length r-haFGF (20 mg) free from other components was eluted after the truncated r-haFGF enriched fractions. The purified r-haFGF was soluble upon dialysis against PBS.

## EXAMPLE 8

### Stabilization

Highly purified and soluble r-haFGF, from Examples 5 and 6 at concentrations of 0.4 mg/ml, 1.2 mg/ml and 2.1 mg/ml were combined with heparin sodium USP (powder, Hepar Industries, Inc.) at a concentration of 0.15 mg/ml. The combined r-haFGF and heparin was stored at either 4°C or 25°C for times ranging from 4 to 7 months and stability and mitogenesis activity were determined. The results are shown in the following table.

TABLE 3

| STABILITY OF SOLUBLE aFGF IN PBS | | | | | |
|---|---|---|---|---|---|
| aFGF (mg.ml) | Stabilizer Heparin (0.15 mg/ml) | Storage Temp. (C°) | Condition Length (Month) | aFGF Stabilitiy % | Mitogenesis Activity $E_{50}^D$ (pg/ml) |
| 0.4 | - | 4 | 7 | 50 | 150 |
| | + | 4 | 7 | >95 | 145 |
| | - | 25 | 5 | 0 | - |
| | + | 25 | 7 | 80 | 182 |
| 1.2 | - | 4 | 6 | 58 | 224 |
| | + | 4 | 6 | >95 | 158 |
| | - | 25 | 6 | 5 | - |
| | + | 25 | 6 | 83 | 200 |
| 2.1 | - | 4 | 5 | 64 | - |
| | + | 4 | 5 | >95 | 158 |
| | - | 25 | 4 | 0 | - |
| | + | 25 | 5 | >95 | 170 |

## Claims

1. A process for obtaining homogeneously pure, biologically active human recombinant acidic fibroblast growth factor from transformed microorganisms which comprises the sequence of steps of:
   a. extraction;
   b. isolation;
   c. affinity chromatography;
   d. preparative reverse phase chromatography; and
   e. solvent exchange.

2. The process of Claim 1 wherein:
   a. the acidic fibroblast growth factor is extracted from transformed microorganisms by mechanical disruption followed by rapid dilution, centrifugation and collection of the supernatant fluid;
   b. isolation comprises ion exchange chromatography on carboxymethyl-sephadex resin with the acidic fibroblast growth factor being eluted with 0.6 M sodium chloride in phosphate buffer with a pH of 7.2;
   c. affinity chromatography on a Heparin-Sepharose column by elution with 1.5 M NaCl at a pH of 7.2;
   d. preparative reversed-phase high performance liquid chromatography is on a Dyanamax $C_4$ column; and
   e. solvent exchange by dialysis against distilled water then a chaotropic agent combined with a reducing agent followed by dialysis against distilled water.

3. A homogeneously pure, biologically active human acidic fibroblast growth factor characterized by having been prepared by the process of Claim 1.

EP 0 408 146 A2

4. The homogeneously pure, biologically active human acidic fibroblast growth factor of Claim 3 wherein the human acidic fibroblast growth factor is soluble in aqueous formulation buffers at concentrations higher than 50 μg/ml.

5. A process for obtaining biologically active, homogeneously pure full-length and truncated recombinant acidic fibroblast growth factor from a combinantion of said growth factors wich comprises hydrophobic interaction chromatography.

6. Full-length recombinant human acidic fibroblast growth factor prepared by the process of claim 5 which is essentially free of truncated recombinant human acidic fibroblast growth factor.

7. Truncated recombinant human acidic fibroblast growth factor prepared by the process of claim 5 which is essentially free of full-length recombinant human acidic fibroblast growth factor.

8. A method of stablizing a soluble, aqueous medicinal composition containing recombinant human acidic fibroblast growth factor as an acitve ingredient, which method comprises incorporation in said composition of an amount of heparin sufficient to stabilize said recombinant human acidic fibroblast growth factor against loss of biological activity.

9. A process for obtaining homogeneously pure, biologically active human recombinant acidic fibroblast growth factor from transformed microorganisms which comprises the sequence of steps of:
  a. extraction;
  b. isolation;
  c. affinity chromatography;
  d. hydrophobic interaction chromatography; and
  e. solvent exchange.

10. The process of Claim 9 wherein:
  a. the acidic fibroblast growth factor is extracted from transformed microorganisms by mechanical disruption followed by rapid dilution, centrifugation and collection of the supernatant fluid;
  b. isolation comprises ion exchange chromatography on carboxymethyl-Sephadex resin with the acidic fibroblast growth factor being eluted with 0.6 M sodium chloride in phosphate buffer with a pH of 7.2;
  c. affinity chromatography on a Heparin-Sepharose column by elution with 1.5 M NaCl at a pH of 7.2;
  d. hydrophobic interaction-high performance liquid chromatography on TSK gel Ether-5PW or Hydropore-HIC with the acidic fibroblast growth factor being eluted with a reverse salt gradient of 2-1 M ammonium sulfate in phosphate buffered saline; and
  e. solvent exchange by dialysis against distilled water then a chaotropic agent combined with a reducing agent then a chaotropic agent and followed by dialysis against distilled water.

11. A homogeneously pure, biologically active human acidic fibroblast growth factor characterized by having been prepared by the process of Claim 9.

12. The homogeneously pure, biologically active human acidic fibroblast growth factor of Claim 11 wherein the human acidic fibroblast growth factor is soluble in aqueous formilation buffers at concentrations higher than 50 μg/ml.

13. A process for obtaining homogeneously pure, biologically active human recombinant acidic fibroblast growth factor from transformed microorganisms which comprises the sequence of steps of:
  a. extraction;
  b. isolation;
  c. affinity chromatography;
  d. hydrophobic interaction chromatography.

14. The process of Claim 11 wherein:
  a. the acidic fibroblast growth factor is extracted from transformed microorganisms by mechanical disruption followed by rapid dilution, centrifugation and collection of the supernatant fluid;
  b. isolation comprises ion exchange chromatography on carboxymethyl-Sephadex resin with the acidic fibroblast growth factor being eluted with 0.6 M sodium chloride in phosphate buffer with a pH of 7.2;
  c. affinity chromatography on a Heparin-Sepharose column by elution with 1.5 M NaCl at a pH of 7.2;
  d. hydrophobic interaction chromatography on Butyl-Agarose or Hexyl-Agarose with the acidic fibroblast growth factor being eluted with a reverse salt gradient of 1.8 to 1 M ammonium sulfate in phosphate buffered saline.

15. A monogeneously pure, biologically active human acidic fibroblast growth factor characterized by having been prepared by the process of Claim 13.

16. The homogeneously pure, biologically active human acidic fibroblast factor of Claim 15 wherein the human acidic fibroblast growth factor is soluble in aqueous formulation buffers at concentrations higher that 50 μg/ml.

9

FIG. 1A

FIG. 1B

FIG. 1C

FIG. 1D